# EUROPEAN PATENT SPECIFICATION

(11) **EP 3 388 421 B1**
(45) Date of publication and mention of the grant of the patent: **04.12.2019**
(21) Application number: 17166501.1
(22) Date of filing: 13.04.2017
(51) Int. Cl.: C07D 239/54, A61K 31/505, A61P 5/02

(54) **ACID ADDITION SALTS OF AN ORALLY AVAILABLE GONADOTROPIN-RELEASING HORMONE RECEPTOR ANTAGONIST**
SÄUREADDITIONSSALZE EINES ORAL VERFÜGBAREN, GONADOTROPINFREISETZENDEN HORMONREZEPTORANTAGONISTEN
SELS D'ADDITION D'ACIDE D'UN ANTAGONISTE RÉCEPTEUR D'HORMONE LIBÉRANT DU GONADOLIBÉRINE DISPONIBLE ORALEMENT

(43) Date of publication of application: 17.10.2018
(73) Proprietor: Sandoz AG, 4056 Basel (CH)
(72) Inventor: MARTIN, Nolwenn, 6250 Kundl (AT); SCHREINER, Erwin, Paul, 6250 Kundl (AT); SILBERBERGER, Herbert, 6250 Kundl (AT)
(74) Representative: Prüfer & Partner mbB Patentanwälte · Rechtsanwälte

(56) References cited:
- WO-A1-2014/143669
- WO-A1-2017/007895
- C. CHEN ET. AL.: "Discovery of Sodium R-(+)-4-{2-[5-(2-Fluoro-3-methoxyphenyl)-3 -(2-fluoro-6-[trifluoromethyl]benzyl)-4-me thyl-2,6-dioxo-3,6-dihydro-2H-pyrimidin-1- yl]-1-phenylethylamino}butyrate (Elagolix), a Potent and Orally Available Nonpeptide Antagonist of the Human Gonadotropin Releasing Hormone Receptor", JOURNAL OF MEDICINAL CHEMISTRY, vol. 51, 11 December 2008 (2008-12-11), pages 7478-7485, XP002770580, DOI: 10.1021/jm8006454

## Description

### FIELD OF THE INVENTION

The invention relates to acid addition salts of elagolix with strong acids, such as sulfuric acid and hydrochloric acid, to processes for their preparation and to a pharmaceutical composition comprising one of said salts, preferably in an effective and/or predetermined amount, wherein the pharmaceutical composition can be used as a medicament, in particular for the treatment of endometriosis and uterine fibroids.

### BACKGROUND OF THE INVENTION

Elagolix is an orally available gonadotropin-releasing hormone (GnRH) receptor antagonist currently under investigation in clinical phase III trials for the treatment of endometriosis and uterine fibroids. The chemical name of elagolix is 4-[[(1*R*)-2-[5-(2-fluoro-3-methoxyphenyl)-3-[[2-fluoro-6-(trifluoromethyl)phenyl]methyl]-3,6-dihydro-4-methyl-2,6-dioxo-1(2*H*)-pyrimidinyl]-1-phenylethyl]amino]butanoic acid. Elagolix is an uracil derivative which can be represented by the chemical structure according to Formula (I)

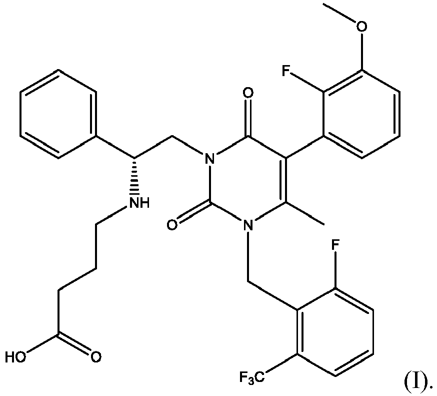

WO 2005/007165 A1 discloses pyrimidine-2,4-dione derivatives as gonadotropin-releasing hormone receptor antagonists. The publication mentions on page 12 that said compounds may generally be used as the free acid or free base or alternatively in form of acid or base addition salts followed by a long list of potentially suitable acids and bases. The compound elagolix is disclosed as one example of pyrimidine-2,4-dione derivatives. In Example 1H of said application elagolix free acid was formed as an intermediate during production of the elagolix sodium salt and was described as a white gel after extraction with ethyl acetate and evaporation of the solvent. The gel was passed through a DOWEX MSC-1 macroporous strong cation-exchange column to convert the acid to the sodium salt. Finally, lyophilization gave the sodium salt of elagolix as a white solid.

According to Chen C. et al. "Discovery of Sodium R-(+)-4-{2-[5-(2-Fluoro-3-methoxyphenyl)-3-(2-fluoro-6-[trifluoromethyl]benzyl)-4-methyl-2,6-dioxo-3,6-dihydro-2H-pyrimidin-1-yl]-1-phenylethylamino}butyrate (Elagolix), a Potent and Orally Available Nonpeptide Antagonist of the Human Gonadotropin-Releasing Hormone Receptor" J. Med. Chem., 2008, 51 (23), 7478-7485 elagolix free acid was either obtained as white foam or as gel after extraction with ethyl acetate and subsequent evaporation of the solvent. As already previously described in WO 2005/007165 A1 the gel was passed through a DOWEX MSC-1 macroporous strong cation-exchange column to convert it to the sodium salt, which was obtained as white solid after lyophilization.

WO 2009/062087 A1 describes processes for the preparation of uracil derivatives, a class of gonadotropin-releasing hormone receptor antagonists including elagolix. Elagolix free acid (Example 4, final product of step 4B) and its sodium salt (Example 5, final product of step 5B and alternate step 5B) were both obtained as off-white solids. On page 6, the application describes the compounds of the application as amorphous solids and goes on to suggest that the compounds of the application are formulated as amorphous cosolutions, for example by spray-drying with excipients such as PVP (polyvinyl pyrrolidone, Kollidons) or HPMC (hydroxypropylmethylcellulose). Amorphous elagolix sodium is described as preferred for that purpose and in Example 9 a solid amorphous mixture was prepared from elagolix sodium and polymers such as HPMC and Kollidon, wherein an excess of polymer at a weight ratio of 1:3 has been used.

The drug substance elagolix is described to be an amorphous hygroscopic solid on page 10 of WO 2017/007895 A1. In Examples 1 and 2 of said application, elagolix drug substance was employed in form of its sodium salt.

Extensive solid-form screening of a new drug is an important step during pharmaceutical development. It is noteworthy that elagolix or its pharmaceutically acceptable salts have not been obtained as crystalline materials so far during pharmaceutical development of elagolix despite what must have been considerable efforts. Even worse, elagolix sodium is highly hygroscopic and was found to deliquesce upon moisture contact. Such properties requiring special care and precautionary measures, such as controlled atmosphere during pharmaceutical processing, which renders manufacturing cumbersome and expensive. The solid dispersion of elagolix sodium with HPMC, which is disclosed in WO 2009/062087 A1, introduces the 3-fold amount of polymer in relation to the active substance, which significantly increases the weight and therefore also the volume of an oral solid dosage form such as a tablet or a capsule. Such formulations may negatively influence patients' compliance, in particular for elderly people having troubles swallowing.

It was thus an objective of the present invention to provide improved solid forms of elagolix, which address some or all of the above-mentioned problems of the elagolix sodium salt.

### SUMMARY OF THE INVENTION

The present invention provides pharmaceutically acceptable non-deliquescent acid addition salts of elagolix with strong acids, such as acids selected from the group consisting of sulfuric acid and hydrochloric acid, wherein the acid addition salt is amorphous or crystalline. It was surprisingly found that salts of elagolix with strong acids, in particular wherein the stoichiometry of elagolix : anion is about 1:1, can be obtained which do not deliquesce and /or show unexpected low hygroscopicity, in particular when compared to the widely used salts of elagolix with a base, like the elagolix sodium salt. They therefore demonstrate physicochemical properties making them suitable for various purposes in the pharmaceutical field, in particular for storage and/or for the preparation of a pharmaceutical composition.

### Abbreviations

- PXRD: powder X-ray diffractogram
- RH: relative humidity
- RT: room temperature
- w-%: weight percent

### Definitions

In the context of the present invention the following definitions have the indicated meaning, unless explicitly stated otherwise:
The term "elagolix" as used herein refers to the compound with the chemical name 4-[[(1*R*)-2-[5-(2-fluoro-3-methoxyphenyl)-3-[[2-fluoro-6-(trifluoromethyl)phenyl]methyl]-3,6-dihydro-4-methyl-2,6-dioxo-1(2*H*)-pyrimidinyl]-1-phenylethyl]amino]butanoic acid, which is represented by the chemical structure as depicted in Formula (I) of the present invention. In the present invention "elagolix" indicates the free acid form, where the hydrogen atom of the carboxylic acid group is not substituted by another kind of atom, for example by sodium or potassium.

As used herein, the term "strong acid" refers to an acidic compound having a pKa in water at a temperature of 25°C of at most 1.0, more preferably of at most 0.0, such as at most -1.0. With regard to the definition of pKa and pharmaceutically acceptable salts of strong acids, reference is made to Stahl, P. H., & Wermuth, C. G. (2002): Handbook of pharmaceutical salts: Properties, selection, and use. Weinheim: Wiley-VCH.

As used herein, the term "an acid addition salt of a compound with sulfuric acid" can be a hydrogen sulfate salt or a sulfate salt of a compound, preferably a sulfate salt of a compound. Thus, when applied to elagolix, the term "an acid addition salt of elagolix with sulfuric acid" can be a hydrogen sulfate salt or a sulfate salt of elagolix, preferably a sulfate salt of elagolix.

As used herein, the term "an acid addition salt of a compound with hydrochloric acid" is a hydrochloride salt of a compound, preferably a monohydrochloride salt of a compound. Thus, when applied to elagolix, the term "an acid addition salt of elagolix with hydrochloric acid" is a hydrochloride salt, preferably a monohydrochloride salt of elagolix.

As used herein the term "room temperature" refers to a temperature in the range of from 20 to 30 °C, preferably to a temperature in the range of from 22 to 27 °C, more preferably to a temperature in the range of from 23 to 26 °C.

As used herein, the term "measured at a temperature in the range of from 20 to 30 °C" refers to a measurement under standard conditions. Typically, standard conditions mean a temperature in the range of from 20 to 30 °C, i.e. at room temperature. A preferred temperature for measurements is 22 °C. Typically, standard conditions additionally mean a measurement at 20% to 75% RH, with about 40% RH being a preferred controlled humidity value for a measurement.

As used herein the term "amorphous" refers to a solid form of a compound that is not crystalline. An amorphous compound possesses no long-range order and does not display a definitive X-ray diffraction pattern with reflections.

The term "reflection" with regards to powder X-ray diffraction as used herein, means peaks in an X-ray diffractogram, which are caused at certain diffraction angles (Bragg angles) by constructive interference from X-rays scattered by parallel planes of atoms in solid material, which are distributed in an ordered and repetitive pattern in a long-range positional order. Such a solid material is classified as crystalline material, whereas amorphous material is defined as solid material, which lacks long-range order and only displays short-range order, thus resulting in broad scattering. According to literature, long-range order e.g. extends over approximately 10³ to 10²⁰ atoms, whereas short-range order is over a few atoms only (see *"*Fundamentals of Powder Diffraction and Structural Characterization of Materials" by Vitalij K. Pecharsky and Peter Y. Zavalij, Kluwer Academic Publishers, 2003, page 3).

The term "essentially the same" with reference to powder X-ray diffraction means that variabilities in peak positions and relative intensities of the peaks need to be taken into account. For example, a typical precision of the 2-Theta values is in the range of ± 0.2° 2-Theta, preferably in the range of ± 0.1° 2-Theta. Thus, a diffraction peak that usually appears at 8.6° 2-Theta for example can appear between 8.4° and 8.8° 2-Theta, preferably between 8.5° and 8.7° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

The term "physical form" or "solid form", which may be used interchangeably herein, refers to any crystalline and amorphous phase of a compound.

A "predetermined amount" as used herein with regard to an acid addition salt of elagolix of the present invention refers to a defined amount of said acid addition salt of elagolix to be used for the preparation of a unit dose of a pharmaceutical composition.

The term "effective amount" as used herein with regard to an acid addition salt of elagolix of the present invention encompasses an amount of said acid addition salt of elagolix, which causes a desired therapeutic and/or prophylactic effect.

An acid addition salt of elagolix may be referred to herein as being characterized by graphical data "as shown in" a figure. Such data include, for example, powder X-ray diffractograms. The person skilled in the art understands that factors such as variations in instrument type, response and variations in sample directionality, sample concentration and sample purity may lead to small variations for such data when presented in graphical form, for example variations relating to the exact peak positions and intensities.

"Impurities" as used herein are organic impurities as defined in the ICH tripartite guideline "Impurities in new drug substances" Q3A(R2), step4 version dated 25.10.2006. In any given sample, this means the organic components of a sample comprising elagolix or an acid addition salt thereof that is not elagolix or an acid addition salt thereof. Briefly, organic impurities can arise during the manufacturing process and/or storage of an active pharmaceutical ingredient. They can include starting materials, by-products, intermediates, degradation products, reagents, ligands and catalysts.

As used herein the term "deliquescent" means the property of a solid form of a compound to readily absorb moisture form the surrounding atmosphere until it deliquesces and forms a solution upon storage for 14 days at 40 °C, atmospheric pressure and 75% relative humidity.

As used herein the term "moderately hygroscopic" refers to a solid form of a compound which shows a water uptake of at most 8.0 w-% in the sorption cycle when measured with gravimetric moisture sorption at a relative humidity in the range of from 0% to 80% and a temperature of 25.0 ± 0.1 °C, based on the weight of the compound.

As used herein, the term "about" means within a statistically meaningful range of a value. Such a range can be within an order of magnitude, typically within 10%, more typically within 5%, even more typically within 1% and most typically within 0.1% of the indicated value or range. Sometimes, such a range can lie within the experimental error, typical of standard methods used for the measurement and/or determination of a given value or range.

### BRIEF DESCRIPTION OF THE DRAWINGS

**Figure 1****:** illustrates a representative PXRD of the crystalline acid addition salt of elagolix with sulfuric acid of the present invention. The x-axis shows the scattering angle in °2-Theta, the y-axis shows the intensity of the scattered X-ray beam in counts of detected photons.
**Figure 2****:** illustrates a representative PXRD of the amorphous acid addition salt of elagolix with hydrochloric acid of the present invention. The x-axis shows the scattering angle in °2-Theta, the y-axis shows the intensity of the scattered X-ray beam in counts of detected photons.

### DETAILED DESCRIPTION OF THE INVENTION

The present invention relates to non-deliquescent acid addition salts of elagolix with strong acids, for example an acid addition salt of elagolix with sulfuric acid or with hydrochloric acid, for example wherein the molar ratio of elagolix and acid is in the range of from 1.0 : 0.8 to 1.0 : 1.2. The acid addition salt of elagolix with a strong acid is amorphous or crystalline. Preferably the acid addition salt of elagolix with a strong acid are only moderately hygroscopic.

The present inventors have found that elagolix can form non-deliquescent acid addition salts with strong acids. This is in contrast to the elagolix salts with bases, such as the elagolix sodium salt, which are deliquescent and take up so much water upon two weeks storage at 40°C and 70% relative humidity that these salts dissolve in that water. The non-deliquescent acid addition salts of the present invention have the advantage that they are particularly suitable for storage and/or for the preparation of a pharmaceutical composition. In addition, the crystalline acid addition salt of elagolix with sulfuric acid of the present invention is a solid form of elagolix, which is more convenient to handle during pharmaceutical processing, for example which is easier to handle during the formulation of a drug product, in particular during the formulation of an oral solid dosage form such as a capsule or a tablet. A further advantage of the crystalline acid addition salt of elagolix with sulfuric acid of the present invention is that it can be used to efficiently deplete impurities from elagolix.

Different aspects of the invention are described below in further detail by embodiments, without being limited thereto. Each aspect of the invention may be described by one embodiment or by combining two or more of the embodiments.

A first aspect of the present invention concerns an acid addition salt of elagolix of Formula (I) 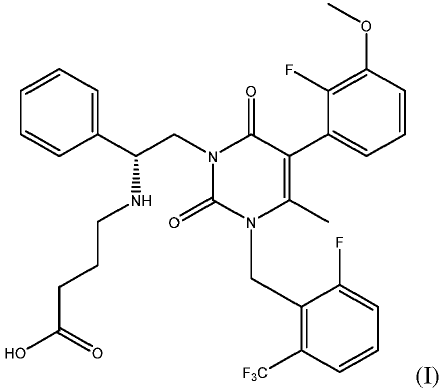 with a strong acid, which acid addition salt is non-deliquescent, wherein the acid addition salt is amorphous or crystalline.

Strong acids which form pharmaceutically acceptable acid addition salts with elagolix are preferred, such as hydrobromic acid, hydrochloric acid, naphthalene-1,5-disulfonic acid, sulfuric acid, ethane-1,2-disulfonic acid, cyclamic acid, p-toluenesulfonic acid, thiocyanic acid, nitric acid, methanesulfonic acid, dodecylsulfuric acid, naphthalene-2-sulfonic acid and benzenesulfonic acid, with hydrobromic acid, hydrochloric acid, sulfuric acid, ethane-1,2-disulfonic acid, p-toluenesulfonic acid and methanesulfonic acid being even more preferred. Acid addition salts which are only moderately hygroscopic are particularly preferred.

In a preferred embodiment the present invention relates to a non-deliquescent salt of elagolix with sulfuric acid, in particular wherein the acid addition salt of elagolix with sulfuric acid is crystalline.

The crystalline acid addition salt of elagolix with sulfuric acid of the present invention may be characterized by analytical methods well known in the field of the pharmaceutical industry for characterizing solids. A preferred method for characterizing said crystalline solid is for example powder X-ray diffraction.

In one embodiment, the present invention relates to the crystalline acid addition salt of elagolix with sulfuric acid characterized by having a PXRD comprising reflections at 2-Theta angles of:
(7.6 ± 0.2)°, (8.6 ± 0.2)° and (10.0 ± 0.2)°; or
(7.6 ± 0.2)°, (8.6 ± 0.2)°, (10.0 ± 0.2)° and (15.5 ± 0.2)°; or
(7.6 ± 0.2)°, (8.6 ± 0.2)°, (10.0 ± 0.2)°, (15.5 ± 0.2)° and (18.1 ± 0.2)°; or
(7.6 ± 0.2)°, (8.6 ± 0.2)°, (10.0 ± 0.2)°, (15.5 ± 0.2)°, (18.1 ± 0.2)° and (20.1 ± 0.2)°; or
(7.6 ± 0.2)°, (8.6 ± 0.2)°, (9.1 ± 0.2)°, (10.0 ± 0.2)°, (15.5 ± 0.2)°, (18.1 ± 0.2)° and (20.1 ± 0.2)°; or
(7.6 ± 0.2)°, (8.6 ± 0.2)°, (9.1 ± 0.2)°, (10.0 ± 0.2)°, (14.2 ± 0.2)°, (15.5 ± 0.2)°, (18.1 ± 0.2)° and (20.1 ± 0.2)°; or
(7.6 ± 0.2)°, (8.6 ± 0.2)°, (9.1 ± 0.2)°, (10.0 ± 0.2)°, (14.2 ± 0.2)°, (15.5 ± 0.2)°, (18.1 ± 0.2)°, (19.0 ± 0.2)° and (20.1 ± 0.2)°; or
(7.6 ± 0.2)°, (8.6 ± 0.2)°, (9.1 ± 0.2)°, (10.0 ± 0.2)°, (14.2 ± 0.2)°, (14.8 ± 0.2)°, (15.5 ± 0.2)°, (18.1 ± 0.2)°, (19.0 ± 0.2)° and (20.1 ± 0.2)°;
when measured at a temperature in the range of from 20 to 30 °C with Cu-Kalpha_{1,2} radiation having a wavelength of 0.15419 nm.

In another embodiment, the present invention relates to the crystalline acid addition salt of elagolix with sulfuric acid characterized by having a PXRD comprising reflections at 2-Theta angles of:
(7.6 ± 0.1)°, (8.6 ± 0.1)° and (10.0 ± 0.1)°; or
(7.6 ± 0.1)°, (8.6 ± 0.1)°, (10.0 ± 0.1)° and (15.5 ± 0.1)°; or
(7.6 ± 0.1)°, (8.6 ± 0.1)°, (10.0 ± 0.1)°, (15.5 ± 0.1)° and (18.1 ± 0.1)°; or
(7.6 ± 0.1)°, (8.6 ± 0.1)°, (10.0 ± 0.1)°, (15.5 ± 0.1)°, (18.1 ± 0.1)° and (20.1 ± 0.1)°; or
(7.6 ± 0.1)°, (8.6 ± 0.1)°, (9.1 ± 0.1)°, (10.0 ± 0.1)°, (15.5 ± 0.1)°, (18.1 ± 0.1)° and (20.1 ± 0.1)°; or
(7.6 ± 0.1)°, (8.6 ± 0.1)°, (9.1 ± 0.1)°, (10.0 ± 0.1)°, (14.2 ± 0.1)°, (15.5 ± 0.1)°, (18.1 ± 0.1)° and (20.1 ± 0.1)°; or
(7.6 ± 0.1)°, (8.6 ± 0.1)°, (9.1 ± 0.1)°, (10.0 ± 0.1)°, (14.2 ± 0.1)°, (15.5 ± 0.1)°, (18.1 ± 0.1)°, (19.0 ± 0.1)° and (20.1 ± 0.1)°; or
(7.6 ± 0.1)°, (8.6 ± 0.1)°, (9.1 ± 0.1)°, (10.0 ± 0.1)°, (14.2 ± 0.1)°, (14.8 ± 0.1)°, (15.5 ± 0.1)°, (18.1 ± 0.1)°, (19.0 ± 0.1)° and (20.1 ± 0.1)°;
when measured at a temperature in the range of from 20 to 30 °C with Cu-Kalpha_{1,2} radiation having a wavelength of 0.15419 nm.

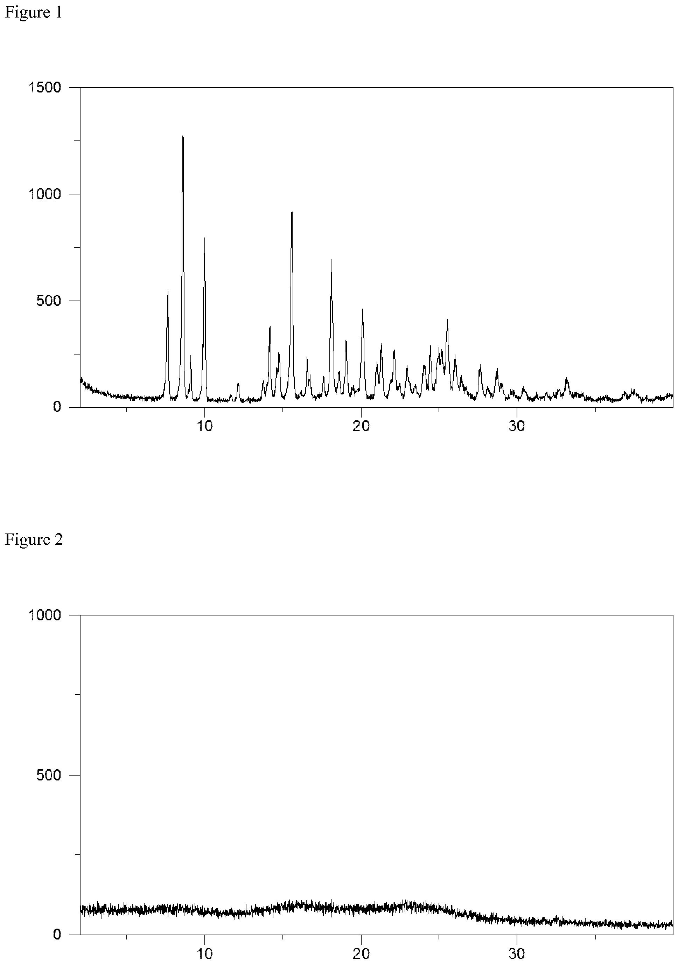

In still another embodiment, the present invention relates to the crystalline acid addition salt of elagolix with sulfuric acid characterized by having a PXRD essentially the same as shown in Figure 1 of the present invention, when measured at a temperature in the range of from 20 to 30 °C with Cu-Kalpha_{1,2} radiation having a wavelength of 0.15419 nm.

In a further aspect, the invention refers to a non-deliquescent acid addition salt of elagolix of Formula (I) with hydrochloric acid.

In a preferred embodiment, the acid addition salt of elagolix with hydrochloric acid is amorphous.

In one embodiment, the acid addition salt of elagolix with hydrochloric acid is characterized by having a powder X-ray diffractogram comprising no reflection in the range of from 2 to 40° 2-Theta, when measured at a temperature in the range of from 20 to 30 °C with Cu-Kalpha_{1,2} radiation having a wavelength of 0.15419 nm.

In a further embodiment, the acid addition salt of elagolix with hydrochloric acid is characterized by having a PXRD essentially the same as shown in Figure 2 of the present invention, when measured at a temperature in the range of from 20 to 30 °C with Cu-Kalpha_{1,2} radiation having a wavelength of 0.15419 nm.

In a particular embodiment, the acid addition salts of elagolix with the strong acid, in particular with a pharmaceutically acceptable acid such as sulfuric acid or hydrochloric acid, is characterized by having a molar ratio of elagolix and acid in the range of from 1.0 : 0.8 to 1.0 : 1.2, preferably of from 1.0 : 0.9 to 1.0 : 1.1 and most preferably the molar ratio is 1.0 : 1.0.

Surprisingly, the acid addition salts of elagolix of the present invention, such as the crystalline acid addition salt of elagolix with sulfuric acid and the amorphous acid addition salt of elagolix with hydrochloric acid, do not deliquesce upon moisture contact but preserve their solid form even under accelerated stress conditions of 40 °C/75% RH. This is advantageous, since there is no need for precautionary measures in order to protect the salts of the present invention during drug product manufacturing from moisture and there is also no need for expensive packaging material, which protects the final drug product comprising the salts of the present invention from humid atmospheres during storage.

In contrast, elagolix sodium and other tested base addition salts of elagolix deliquesced fairly quickly, when they were subjected to the above described accelerated stress conditions. Said property requires moisture protection during pharmaceutical processing, special formulation strategies, such as solid dispersions, thereby adding further excipients to the formulation and making the so formulated pharmaceutical dosage form larger than necessary or desired, and/or special packaging for storage. The results of the accelerated stress test are summarized in Example 2.

In a further aspect, the present invention relates to a process for the preparation of the elagolix acid addition salts as defined above, said process comprising the steps of:
(i) reacting elagolix with a strong acid, such as selected from the group consisting of sulfuric acid or hydrochloric acid, in the presence of an organic solvent and
(ii) separating the elagolix acid addition salt obtained in step (i) from the reaction mixture.

Elagolix provided in step (i) can be prepared according to the teaching of WO 2009/062087 A1 Example 4 yielding amorphous material.

A suitable organic solvent, in which the acid base reaction of step (i) may be carried out is selected from the group consisting of acetonitrile, C₃-C₅ ketones, C₁-C₂ halogenated hydrocarbons, C₃-C₄ alcohols, C₂-C₆ ethers, C₃-C₅ esters or a combination of two or more thereof. Preferably, the solvent is selected from the group consisting of dichloromethane, chloroform, tetrahydrofuran, 2-methyltetrahydrofuran, 2-propanol, ethyl acetate, isopropyl acetate, acetone, acetonitrile or mixtures of two or more thereof. More preferably, the solvent is selected from the group consisting of ethyl acetate, acetonitrile or a mixture thereof. Even more preferably the reaction mixture comprises one or more of the above listed organic solvent(s) and additionally water. Most preferably, the reaction mixture comprises acetonitrile and water or ethyl acetate and water.

The temperature of the reaction mixture is kept in the range of from 0 to 30 °C, more preferably in the range of from 2 to 25 °C, for example in the range of from 20 to 25 °C and preferably the reaction is carried out at ambient pressure. The acid may be applied as aqueous solution containing 37 to 97 w-% acid as described in Example 1 herein. The molar ratio of elagolix and acid applied is in the range of from 1.0 : 0.8 to 1.0 : 1.2, preferably of from 1.0 : 0.9 to 1.0 : 1.1 and most preferably the molar ratio is 1.0 : 1.0.

The reaction mixture obtained in step (i) is stirred, shaken or allowed to stand at a temperature in the range of from 0 to 30 °C, preferably in the range of from 2 to 25 °C, for example in the range of from 20 to 25 °C, typically for a period of time in the range of from 0.1 to 24 hours in order to promote the formation of the elagolix acid addition salt. Once the reaction is complete, the acid addition salt may remain in solution, precipitate as amorphous material or crystallize.

If the acid addition salt remains in solution it may be separated from the reaction mixture by removing the solvent for example by lyophilization, spray drying, vacuum drying or evaporation. In a spray drying process, the solution is pumped through an atomizer into a drying chamber thereby removing the solvent to form the solid acid addition salt. A drying process uses hot gases, such as air, nitrogen, nitrogen-enriched air or argon, to dry the particles. The solution can be atomized by conventional means well known in the art, such as a two-fluid sonication nozzle and a two-fluid non-sonication nozzle. Preferably, the solvent is removed by lyophilization or spray drying. If the solvent is removed by lyophilization, the sample temperature during lyophilization may be varied or held essentially constant and is preferably in the range of from 20 to 40 °C, more preferably in the range of from 25 to 35 °C.

If the acid addition salt precipitates as amorphous material or crystallizes, the obtained suspension can optionally be further kept at a temperature in the range of from 0 to 20 °C, preferably in the range of from 2 to 10 °C to increase the process yield before the acid addition salt is separated from the reaction mixture by any conventional method such as filtration or centrifugation, most preferably by filtration. Optionally, the isolated solid material may be washed with a solvent. Preferably, the solvent comprises ethyl acetate and most preferably, the solvent is ethyl acetate. Finally, the elagolix acid addition salt may optionally be dried at a temperature in the range of from 20 to 60 °C, more preferably of from 20 to 40 °C and most preferably the elagolix acid addition salt is dried at RT. Drying may be performed for a period of time in the range of from about 1 to 72 hours, preferably from about 2 to 48 hours, more preferably from about 4 to 24 hours and most preferably from about 6 to 18 hours. Drying may be performed at ambient pressure and/or under vacuum preferably at about 100 mbar or less, more preferably at about 50 mbar or less and most preferably at about 30 mbar or less.

In order to promote the crystallization of the acid addition salt of elagolix with sulfuric acid, seed crystals of acid addition salt of elagolix with sulfuric acid as defined hereinabove can optionally be added to the reaction mixture of step (i). Seed crystals can be prepared according to Example 1.1 disclosed herein. The amount of seed crystals employed may range from about 0.1 to 20 w-%, preferably from about 0.5 to 10 w-% and most preferably from about 1 to 5 w-%, based on the weight of applied elagolix starting material.

As already mentioned above, the crystalline acid addition salt of elagolix with sulfuric acid of the present invention efficiently depletes impurities from elagolix and is obtained in high chemical purity. Hence, the crystalline acid addition salt of elagolix with sulfuric acid may also be used as a means for purification of elagolix and may be further used for the preparation of a base addition salt of elagolix.

Therefore, in a further aspect, the invention relates to the use of the crystalline acid addition salt of elagolix with sulfuric acid as described herein for the preparation of a pharmaceutically acceptable base addition salt of elagolix. In a particular embodiment the pharmaceutically acceptable base addition salt is selected from the group consisting of elagolix sodium, potassium, magnesium, calcium, lithium, zinc, ammonium, ethylenediamine, meglumine and lysine. Preferably, the pharmaceutically acceptable base addition salt is selected from elagolix sodium and elagolix potassium, most preferably it is elagolix sodium

In a further aspect, the invention relates to a method for the preparation of a pharmaceutically acceptable base addition salt of elagolix, said method comprising the steps of:
(a) providing an acid addition salt of elagolix with a strong acid, such as the acid addition salt of elagolix with sulfuric acid as described herein,
(b) converting the acid addition salt to elagolix of Formula (I),
(c) reacting elagolix obtained in step (b) with a pharmaceutically acceptable base, such as sodium hydroxide, in the presence of an organic solvent and
(d) separating the pharmaceutically acceptable base addition salt of elagolix, such as elagolix sodium, obtained in step (c) from the reaction mixture.

In another aspect, the present invention relates to the non-deliquescent acid addition salts of elagolix with a strong acid, such as sulfuric acid or hydrochloric acid, as described herein for the preparation of a pharmaceutical composition.

In another aspect, the present invention relates to a non-deliquescent amorphous acid addition salt of elagolix with a strong acid, such as sulfuric acid or hydrochloric acid, for the preparation of a pharmaceutical composition.

In another aspect, the present invention relates to a non-deliquescent crystalline acid addition salt of elagolix with a strong acid, such as sulfuric acid or hydrochloric acid, for the preparation of a pharmaceutical composition.

In a further aspect, the present invention relates to a pharmaceutical composition comprising one of the non-deliquescent acid addition salts of elagolix with a strong acid, such as sulfuric acid or hydrochloric acid as defined herein, preferably in a predetermined and/or effective amount and one or more pharmaceutically acceptable excipient(s). The acid addition salt of elagolix with a strong acid is amorphous or crystalline.

In a preferred embodiment, the predetermined and/or effective amount of the acid addition salt of elagolix of the pharmaceutical composition as defined above is selected from the group consisting of 25 mg, 50 mg, 75 mg, 100 mg, 125 mg, 150 mg, 175 mg, 200 mg, 225 mg, 250 mg, 275 mg and 300 mg calculated as elagolix.

In a further preferred embodiment, the one or more pharmaceutically acceptable excipient(s) of the pharmaceutical composition as defined above is/are one or more diluent(s). Preferably, the one or more diluent(s) is/are selected from the group consisting of carbohydrates such as sugars, sugar alcohols, starches and celluloses. In another embodiment, the sugar may be selected from the group consisting of lactose, e.g. lactose monohydrate, anhydrous lactose or spray dried lactose, sucrose, dextrose, fructose, glucose, maltose and maltodextrin. In still another embodiment, the sugar alcohol may be selected from the group consisting of mannitol, sorbitol, xylitol and inositol. In a further embodiment the starches may be selected from corn starch and potato starch, whereas the starches are preferably pre-gelatinized or hydrolyzed. In yet another embodiment the celluloses may be selected from the group consisting of powdered cellulose, microcrystalline cellulose and silicified cellulose. In another preferred embodiment the one or more diluent(s) may also be selected from inorganic materials such as but not limited to calcium phosphate, calcium carbonate, calcium sulfate, calcium lactate, sodium chloride, magnesium oxide and magnesium carbonate.

In still another preferred embodiment, the pharmaceutical composition of the present invention is an oral solid dosage form such as a tablet or a capsule and most preferably the pharmaceutical composition is a tablet.

The pharmaceutical composition as defined above may be prepared by pharmaceutical standard procedures e.g. by wet or dry processing methods. In certain embodiments the pharmaceutical composition is prepared by wet processing methods, such as, but not limited to, wet granulation methods. Suitable wet granulation methods comprise high-shear granulation or fluid bed granulation. In another embodiment the pharmaceutical composition is prepared by dry processing methods, such as, but not limited to, direct compression or dry granulation methods. An example of dry granulation is roller compaction. The pharmaceutical composition obtained by dry or wet processing methods are preferably compressed into tablets or encapsulated.

The present invention also relates to the pharmaceutical composition as defined above for use as a medicament.

The present invention also relates to the pharmaceutical composition as defined above for the treatment of endometriosis and uterine fibroids.

The present inventors have found that acid addition salts of elagolix with strong acids, such as hydrochloric acid or sulfuric acid, can be obtained as solid forms which are non-deliquescent. This is contrary to the base addition salts which are widely used for elagolix. This makes acid addition salts of elagolix with strong acids, such as hydrochloric acid or sulfuric acid, attractive for storage and/or shipment, for example as an intermediate for elagolix sodium production.

The present invention therefore also relates to the use of an acid addition salt of elagolix with a strong acid, such as hydrochloric acid or sulfuric acid, for shipment and/or storage.

The present invention also relates to a method for the transport of elagolix, wherein an acid addition salt of elagolix with a strong acid, such as hydrochloric acid or sulfuric acid, is moved from one place to another. Transport can mean transport from one reaction vessel to another. But in particular transport means long-distance transport, where an acid addition salt of elagolix with a strong acid is transported for a distance of more than one kilometer, or even shipped between countries and/or continents.

### EXAMPLES

The following analytical method and parameters have been applied for the generation of the powder X-ray data disclosed in the present invention:

### Powder X-ray diffraction

Powder X-ray diffraction was performed with a PANalytical X'Pert PRO diffractometer equipped with a theta/theta coupled goniometer in transmission geometry, Cu-Kalpha_{1,2} radiation (wavelength 0.15419 nm) with a focusing mirror and a solid state PIXcel detector. Diffractograms were recorded at a tube voltage of 45 kV and a tube current of 40 mA, applying a stepsize of 0.013° 2-Theta with 40s per step (255 channels) in the angular range of 2° to 40° 2-Theta at ambient conditions.

### Gravimetric Moisture Sorption

Moisture sorption and desorption curves are recorded with an SPSx-1µ moisture sorption analyzer (ProUmid, Ulm).

The measurement cycle is started at ambient relative humidity (RH) of 30%. RH is then decreased to 0% in 5% steps. Afterwards RH is increased from 0% to 80% in a sorption cycle.

The time per step is set to a minimum of 2 hours and a maximum of 6 hours. If an equilibrium condition with a constant mass of ± 0.01% within 1 hour is reached before the maximum time for all examined samples the sequential humidity step is applied before the maximum time of 6 hours. If no equilibrium is achieved the consecutive humidity step is applied after the maximum time of 6 hours. The temperature is 25 ± 0.1 °C.

The following non-limiting examples are illustrative for the disclosure and shall not limit the scope of the invention.

### Example 1: Preparation of elagolix acid addition salts

### Example 1.1: Crystalline acid addition salt of elagolix with sulfuric acid

Amorphous elagolix (200 mg, for example prepared according to the procedure disclosed in WO 2009/062087 A1, example 4B) was dissolved in 3.0 mL ethyl acetate, followed by filtration with the aid of a syringe filter (pore size 0.45 microns). Sulfuric acid (95-97 w-% aqueous solution, 1.05 mol equivalent, 18.8 microliter) was diluted with 1.0 mL ethyl acetate and added dropwise to the elagolix solution under stirring. The thus obtained mixture was further stirred at room temperature for 18 hours, leading to the formation of a homogeneous suspension. The solid material was collected by filtration, washed with a little amount of ethyl acetate and dried under vacuum (30 mbar) at room temperature for 16 hours, yielding the acid addition salt of elagolix with sulfuric acid as a white solid. The obtained material was crystalline as confirmed by the PXRD depicted in Figure 1 herein. The corresponding reflection list is provided in Table 1 below.

**Table 1: PXRD reflections and corresponding relative intensities of the crystalline acid addition salt of elagolix with sulfuric acid prepared according to Example 1.1 in the range of from 2 to 30° 2-Theta; A typical precision of the 2-Theta values is in the range of ± 0.2° 2-Theta, preferably of ± 0.1° 2-Theta.**

| Angle [°2-Theta] | Relative Intensity [%] | Angle [°2-Theta] | Relative Intensity [%] |
|---|---|---|---|
| 7.6 | 41 | 21.0 | 13 |
| 8.6 | 100 | 21.3 | 20 |
| 9.1 | 17 | 22.1 | 15 |
| 10.0 | 62 | 22.5 | 5 |
| 11.7 | 2 | 22.9 | 11 |
| 12.1 | 6 | 23.5 | 4 |
| 13.8 | 7 | 24.1 | 11 |
| 14.2 | 28 | 24.4 | 19 |
| 14.8 | 18 | 25.0 | 17 |
| 15.5 | 71 | 25.2 | 17 |
| 16.5 | 16 | 25.5 | 27 |
| 16.7 | 8 | 26.0 | 14 |
| 17.6 | 6 | 26.4 | 7 |
| 18.1 | 48 | 27.6 | 13 |
| 18.6 | 10 | 28.1 | 4 |
| 19.0 | 21 | 28.7 | 9 |
| 19.5 | 3 | 29.0 | 6 |
| 20.1 | 33 | 29.7 | 4 |

### Example 1.2: Amorphous acid addition salt of elagolix with hydrochloric acid

Amorphous elagolix (300 mg, for example prepared according to the procedure disclosed in WO 2009/062087 A1, example 4B) was dissolved in 4.2 mL acetonitrile/water (volume ratio 1:1). Hydrochloric acid fuming (37 w-% aqueous solution, 1.0 mol equivalent, 39.4 microliter) was diluted with 1.5 mL acetonitrile/water (volume ratio 1:1) and added to the elagolix solution. The thus obtained solution was shaken at room temperature and afterwards allowed to stand without shaking for about 10 min before it was filtered with the aid of a syringe filter (pore size 0.45 microns). The homogeneous solution was frozen in a bath of liquid nitrogen and lyophilized at room temperature and a pressure below 2 mbar, yielding acid addition salt of elagolix with hydrochloric acid as a white solid. The obtained material was amorphous as confirmed by the PXRD depicted in Figure 2 herein.

### Example 2: Accelerated stress test at 40 °C/ 75% RH with elagolix acid addition salts vs. base addition salts

The physical stability of different elagolix salts against moisture and temperature stress has been tested. For this purpose, the elagolix salts were open stored at accelerated stress conditions of 40 °C and 75% RH for 1, 3 and 8 weeks respectively. The physical stability has been investigated by means of powder X-ray diffraction and the consistency of the samples was visually controlled. The results are summarized in Table 2 below.

**Table 2: Results of comparative stress test with different elagolix salts**

| **Salts of elagolix with** | **Initial sample** | **1 week at 40°C/75% RH** | **3 weeks at 40°C/75% RH** | **8 weeks at 40°C/75% RH** |
|---|---|---|---|---|
| sulfuric acid HCl | crystalline solid amorphous solid | crystalline solid amorphous solid | crystalline solid amorphous solid | crystalline solid amorphous solid |
| NaOH | amorphous solid | deliquescence | - | - |
| KOH | amorphous solid | deliquescence | - | - |
| L-arginine | amorphous solid | deliquescence | - | - |
| choline | amorphous solid | deliquescence | - | - |
| meglumine | amorphous solid | deliquescence | - | - |
| L-lysine | amorphous solid | deliquescence | - | - |

As can be seen from Table 2, those acid addition salts of elagolix prepared with strong acids, such as sulfuric acid or hydrochloric acid remained solid throughout the whole stress test. In addition, these salts did not undergo any solid form transformations. The amorphous acid addition salt of elagolix with hydrochloric acid remained amorphous and the crystalline acid addition salt of elagolix with sulfuric acid preserved its crystal structure, which was confirmed by the unchanged powder X-ray diffractograms measured before and after the stress test.

In stark contrast, all tested salts of elagolix with a base including elagolix sodium deliquesced fairly quickly, when they were subjected to the above described stress conditions. Consequently, the stress test for these salts was already discontinued at the first check point after one week.

## Claims

1. A non-deliquescent acid addition salt of elagolix, wherein the acid is a strong acid having a pKa in water of at most 1.0, which acid addition salt is crystalline or amorphous.

2. The acid addition salt of elagolix according to claim 1, **characterized by** having a molar ratio of elagolix : acid in the range of from 1.0 : 0.8 to 1.0 : 1.2.

3. The acid addition salt of elagolix according to claim 1 or claim 2, wherein the acid is selected from the group consisting of hydrobromic acid, hydrochloric acid, naphthalene-1,5-disulfonic acid, sulfuric acid, ethane-1,2-disulfonic acid, cyclamic acid, p-toluenesulfonic acid, thiocyanic acid, nitric acid, methanesulfonic acid, dodecylsulfuric acid, naphthalene-2-sulfonic acid and benzenesulfonic acid.

4. The acid addition salt of elagolix according to any one of claims 1 to 3, wherein the acid is selected from the group consisting of sulfuric acid and hydrochloric acid.

5. The acid addition salt of elagolix with sulfuric acid of claim 4, which is crystalline.

6. The crystalline acid addition salt of elagolix with sulfuric acid according to claim 5 **characterized by** having a powder X-ray diffractogram comprising reflections at 2-Theta angles of:
(7.6 ± 0.2)°, (8.6 ± 0.2)° and (10.0 ± 0.2)°; or
(7.6 ± 0.2)°, (8.6 ± 0.2)°, (10.0 ± 0.2)° and (15.5 ± 0.2)°; or
(7.6 ± 0.2)°, (8.6 ± 0.2)°, (10.0 ± 0.2)°, (15.5 ± 0.2)° and (18.1 ± 0.2)°;
when measured at a temperature in the range of from 20 to 30 °C with Cu-Kalpha_{1,2} radiation having a wavelength of 0.15419 nm.

7. The acid addition salt of elagolix with hydrochloric acid of claim 4, which is amorphous.

8. The acid addition salt of elagolix with hydrochloric acid of claim 4 or 7, which is **characterized by** having a powder X-ray diffractogram comprising no reflection in the range of from 2 to 40° 2-Theta, when measured at a temperature in the range of from 20 to 30 °C with Cu-Kalpha_{1,2} radiation having a wavelength of 0.15419 nm.

9. A process for the preparation of the acid addition salt of elagolix according to any one of the preceding claims comprising:
(i) reacting elagolix with a strong acid in the presence of an organic solvent and
(ii) separating the elagolix acid addition salt obtained in step (i) from the reaction mixture.

10. The process according to claim 9, wherein the reaction mixture further contains water.

11. Use of the acid addition salt of elagolix according to any one of claims 1 to 8 for the preparation of a base addition salt of elagolix.

12. The use according to claim 11 wherein the base addition salt of elagolix is elagolix sodium.

13. Use of the acid addition salt of elagolix according to any one of claims 1 to 8 for the preparation of a pharmaceutical composition.

14. A pharmaceutical composition comprising the acid addition salt of elagolix according to any one of claims 1 to 8 and one or more pharmaceutically acceptable excipient(s).

15. The acid addition salt of elagolix as defined in any one of claims 1 to 8 or the pharmaceutical composition of claim 14 for use as a medicament, in particular for use in the treatment of endometriosis and uterine fibroids.

## Patentansprüche

1. Nicht-zerfließendes Elagolix-Säureadditionssalz, wobei die Säure eine starke Säure mit einem pKa in Wasser von höchstens 1,0 ist, wobei das Säureadditionssalz kristallin oder amorph ist.

2. Elagolix-Säureadditionssalz nach Anspruch 1, **gekennzeichnet durch** ein Molverhältnis von Elagolix : Säure im Bereich von 1,0 : 0,8 bis 1,0 : 1,2.

3. Elagolix-Säureadditionssalz nach Anspruch 1 oder Anspruch 2, wobei die Säure ausgewählt ist aus der Gruppe bestehend aus Bromwasserstoffsäure, Salzsäure, Naphthalen-1,5-disulfonsäure, Schwefelsäure, Ethan-1,2-disulfonsäure, Cyclaminsäure, p-Toluolsulfonsäure, Thiocyanidsäure, Salpetersäure, Methansulfonsäure, Dodecylsulfursäure, Naphthalen-2-sulfonsäure und Benzolsulfonsäure.

4. Elagolix-Säureadditionssalz nach irgendeinem der Ansprüche 1 bis 3, wobei die Säure ausgewählt ist aus der Gruppe bestehend aus Schwefelsäure und Salzsäure.

5. Elagolix-Säureadditionssalz mit Schwefelsäure nach Anspruch 4, das kristallin ist.

6. Kristallines Elagolix-Säureadditionssalz mit Schwefelsäure nach Anspruch 5, **gekennzeichnet durch** ein Pulver-Röntgendiffraktogramm, das Reflexionen aufweist bei 2-Theta-Winkeln von:
(7,6 ± 0,2)°, (8,6 ± 0,2)° und (10,0 ± 0,2)°; oder
(7,6 ± 0,2)°, (8,6 ± 0,2)°, (10,0 ± 0,2)° und (15,5 ± 0,2)°; oder
(7,6 ± 0,2)°, (8,6 ± 0,2)°, (10,0 ± 0,2)°, (15,5 ± 0,2)° und (18,1 ± 0,2)°;
gemessen bei einer Temperatur im Bereich von 20 bis 30 °C mit Cu-Kalpha_{1,2}-Strahlung mit einer Wellenlänge von 0,15419 nm.

7. Elagolix-Säureadditionssalz mit Salzsäure nach Anspruch 4, das amorph ist.

8. Elagolix-Säureadditionssalz mit Salzsäure nach Anspruch 4 oder 7, **dadurch gekennzeichnet, dass** es ein Pulver-Röntgendiffraktogramm aufweist, das keine Reflexion im Bereich von 2 bis 40° 2-Theta umfasst, gemessen bei einer Temperatur im Bereich von 20 bis 30 °C mit Cu-Kalpha_{1,2}-Strahlung mit einer Wellenlänge von 0,15419 nm.

9. Verfahren zur Herstellung des Elagolix-Säureadditionssalzes nach irgendeinem der vorhergehenden Ansprüche, umfassend:
(i) Reagieren von Elagolix mit einer starken Säure in Gegenwart eines organischen Lösungsmittels, und
(ii) Abtrennen des in Schritt (i) erhaltenen Elagolix-Säureadditionssalzes aus dem Reaktionsgemisch.

10. Verfahren nach Anspruch 9, wobei das Reaktionsgemisch ferner Wasser enthält.

11. Verwendung des Elagolix-Säureadditionssalzes nach irgendeinem der Ansprüche 1 bis 8 zur Herstellung eines Elagolix-Basenadditionssalzes.

12. Verwendung nach Anspruch 11, wobei das Elagolix-Basenadditionssalz Elagolix Natrium ist.

13. Verwendung des Elagolix-Säureadditionssalzes nach irgendeinem der Ansprüche 1 bis 8 zur Herstellung einer pharmazeutischen Zusammensetzung.

14. Pharmazeutische Zusammensetzung, umfassend das Elagolix-Säureadditionssalz nach irgendeinem der Ansprüche 1 bis 8 und einen oder mehrere pharmazeutisch akzeptable(n) Hilfsstoff(e).

15. Elagolix-Säureadditionssalz wie in irgendeinem der Ansprüche 1 bis 8 definiert oder die pharmazeutische Zusammensetzung nach Anspruch 14 zur Verwendung als Medikament, insbesondere zur Verwendung bei der Behandlung von Endometriose und Uterusmyomen.

## Revendications

1. Sel d'addition d'acide non déliquescent d'élagolix, dans lequel l'acide est un acide fort présentant un pKa dans l'eau d'au plus 1,0, lequel sel d'addition d'acide est cristallin ou amorphe.

2. Sel d'addition d'acide d'élagolix selon la revendication 1, **caractérisé par** la présence d'un rapport molaire d'élagolix : acide situé dans la plage allant de 1,0 : 0,8 à 1,0 : 1,2.

3. Sel d'addition d'acide d'élagolix selon la revendication 1 ou la revendication 2, dans lequel l'acide est choisi parmi le groupe se composant de l'acide bromhydrique, l'acide chlorhydrique, l'acide naphtalène-1,5-disulfonique, l'acide sulfurique, l'acide éthane-1,2-disulfonique, l'acide cyclamique, l'acide p-toluène sulfonique, l'acide thiocyanique, l'acide nitrique, l'acide méthanesulfonique, l'acide dodécylsulfurique, l'acide naphtalène-2-sulfonique et l'acide benzènesulfonique.

4. Sel d'addition d'acide d'élagolix selon l'une quelconque des revendications 1 à 3, dans lequel l'acide est choisi parmi le groupe se composant de l'acide sulfurique et de l'acide chlorhydrique.

5. Sel d'addition d'acide d'élagolix avec l'acide sulfurique selon la revendication 4, qui est cristallin.

6. Sel d'addition d'acide cristallin d'élagolix avec l'acide sulfurique selon la revendication 5 **caractérisé en ce qu'**il présente un diffractogramme de rayons X sur poudre comprenant des reflets à des angles de 2-thêta de :
(7,6 ± 0,2)°, (8,6 ± 0,2)° et (10,0 ± 0,2)° ; ou
(7,6 ± 0,2)°, (8,6 ± 0,2)°, (10,0 ± 0,2)° et (15,5 ± 0,2)° ; ou
(7,6 ± 0,2)°, (8,6 ± 0,2)°, (10,0 ± 0,2)°, (15,5 ± 0,2)° et (18,1 ± 0,2)° ;
lorsqu'ils sont mesurés à une température située dans la plage allant de 20 à 30 °C avec le rayonnement Cu-Kalpha_{1,2} présentant une longueur d'onde de 0,15419 nm.

7. Sel d'addition d'acide d'élagolix avec l'acide chlorhydrique selon la revendication 4, qui est amorphe.

8. Sel d'addition d'acide d'élagolix avec l'acide chlorhydrique selon la revendication 4 ou 7, qui est **caractérisé par** la réalisation d'un diffractogramme de rayons X sur poudre ne comprenant aucun reflet à des angles 2-thêta situés dans la plage allant de 2 à 40°, lorsqu'ils sont mesurés à une température située dans la plage allant de 20 à 30 °C avec le rayonnement Cu-Kalpha_{1,2} présentant une longueur d'onde de 0,15419 nm.

9. Procédé de préparation du sel d'addition d'acide d'élagolix selon l'une quelconque des revendications précédentes comprenant :
(i) la mise en réaction d'élagolix avec un acide fort en présence d'un solvant organique et
(ii) la séparation du sel d'addition d'acide d'élagolix obtenu à l'étape (i) à partir du mélange réactionnel.

10. Procédé selon la revendication 9, dans lequel le mélange réactionnel contient en outre de l'eau.

11. Utilisation du sel d'addition d'acide d'élagolix selon l'une quelconque des revendications 1 à 8 pour la préparation d'un sel d'addition de base d'élagolix.

12. Utilisation selon la revendication 11 dans laquelle le sel d'addition de base d'élagolix est du sodium d'élagolix.

13. Utilisation du sel d'addition d'acide d'élagolix selon l'une quelconque des revendications 1 à 8 pour la préparation d'une composition pharmaceutique.

14. Composition pharmaceutique comprenant du sel d'addition d'acide d'élagolix selon l'une quelconque des revendications 1 à 8 et un ou plusieurs excipients pharmaceutiquement acceptables.

15. Sel d'addition d'acide d'élagolix tel que défini selon l'une quelconque des revendications 1 à 8 ou composition pharmaceutique selon la revendication 14 destinée à être utilisée comme un médicament, en particulier destinée à une utilisation dans le traitement de l'endométriose et des fibromes utérins.
